# EUROPEAN PATENT SPECIFICATION

(11) **EP 3 170 809 B1**
(45) Date of publication and mention of the grant of the patent: **03.04.2019**
(21) Application number: 15822780.1
(22) Date of filing: 06.01.2015
(51) Int. Cl.: C07D 211/42

(54) **METHOD FOR RECYCLING CHIRAL 1-BENZYL-3-HYDROXYPIPERIDINE BY RACEMIZATION**
VERFAHREN ZUM RECYCLING CHIRALEM 1-BENZYL-3-HYDROXYPIPERIDIN DURCH RACEMISIERUNG
PROCÉDÉ POUR LE RECYCLAGE DE 1-BENZYL-3-HYDROXYPIPÉRIDINE CHIRALE PAR RACÉMISATION

(30) Priority: 16.07.2014 CN 201410337504
(43) Date of publication of application: 24.05.2017
(73) Proprietor: ABA Chemicals Corporation, Taicang City, Jiangsu 215433 (CN)
(72) Inventor: LIN, Zhigang, Taicang City Jiangsu 215433 (CN); JIANG, Yueheng, Taicang City Jiangsu 215433 (CN); CAI, Tong, Taicang City Jiangsu 215433 (CN)
(74) Representative: Proi World Intellectual Property GmbH
(86) International application number: PCT/CN2015/070184
(87) International publication number: WO 2016/008285

(56) References cited:
- EP-A1- 3 115 358
- WO-A1-2013/076630
- CN-A- 1 280 981
- CN-A- 101 284 789
- JP-A- H10 237 069

## Description

### Technical Field

The Invention relates to a field of compounds synthesis, particularly to a racemization recycling method for chiral 1-benzyl-3-hydroxy piperidine.

### Background Art

Chiral 1-benzyl-3-hydroxy piperidine is an important pharmaceutical intermediate. The chiral 1-benzyl-3-hydroxy piperidine is mostly prepared through chiral synthesis or chemical resolution method in prior art. The chemical resolution method has a problem of recycling the chiral hydroxyl of non-target configuration, which is normally recycled through an indirect racemization method. Specifically, the indirect racemization method is to oxidize hydroxyl to ketone and then reduce it to alcohol with sodium borohydride. However, the above method has many defects, for example, the process is complicated and the yield is low. Therefore, an improved method for recycling chiral 1-benzyl-3-hydroxy piperidine is needed to be presented.

### Summary of the Invention

The Invention is to provide a new method for racemization recycling of chiral 1-benzyl-3-hydroxy piperidine, so as to solve the problem in chemical resolution method that the chiral hydroxyl of non-target configuration is difficult to be recycled and the process of indirect racemization method is complicated and the yield is low. With clean reaction, the method, which has not been reported ever before, is suitable for industrialized mass production.

The Invention uses the following technical schemes to achieve the above purpose:
A method for racemization recycling of chiral N-benzyl-3-hydroxy piperidine; it uses chiral 1-benzyl-3-hydroxy piperidine as the raw material to generate racemized products through racemization reaction under the action of strong alkali and high temperature.

The above preparation method is expressed by a chemical equation as follows:

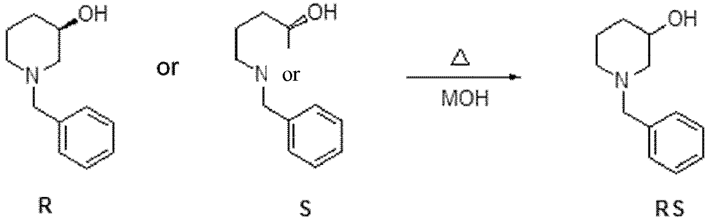

Wherein, the said chiral N-benzyl-3-hydroxy piperidine is (R)-N-benzyl-3-hydroxy piperidine, (S)-N-benzyl-3-hydroxy piperidine or a mixture of the two.

The ee value scope of (R)- N-benzyl-3-hydroxy piperidine and (S)-N-benzyl-3-hydroxy piperidine in the mixture is 0-100%.

In the method of the Invention, the said strong alkali comprises sodium hydroxide, potassium hydroxide or lithium hydroxide, and potassium hydroxide is preferred. This kind of strong alkali can accelerate the racemization.

In the Invention, the temperature for the racemization reaction is 120-300°C, preferably 180-200°C; the reaction time is 1-100 hours, preferably 10-20 hours and most preferably 15 hours. Under such conditions, the reaction can be fully completed with favorable reaction cost and efficiency.

In the method of the Invention, the mole ratio between the said chiral N-benzyl-3-hydroxy piperidine and the strong alkali is 1:1 - 1:5, preferably 1:2.5-1:4 and most preferably 1:2.54. Within the scope, the racemization reaction can occur effectively and fully.

In addition, the method of the Invention also comprises post-processing processes of the racemization reaction as: cooling the reaction liquid to 50°C after racemization reaction; adding water by drops for dilution; dissolving the product through MTBE feeding; cooling the reaction liquid to ambient temperature; separating the liquid; extracting the aqueous phase for one time with MTBE supplement; combing the organic phase and washing it with a small amount of water; obtaining racemized N-benzyl-3-hydroxy piperidine after concentration.

With the above technical scheme, the Invention obtains a brand new method for racemization recycling of chiral 3-hydroxy piperidine, which can obtain a high quality racemized N-benzyl-3-hydroxy piperidine in bulk steadily with safe and high-efficient reaction; isomer can be recycled easily with less three wastes (waste gas, waste water and industrial residues).

### Detailed Description of the Preferred Embodiments

The following detailed embodiments are further descriptions of the technical schemes of the Invention, but the Invention is not limited to this.

### Embodiment 1 Racemization of (R)- N-benzyl-3-hydroxy piperidine (I)

Adding 95.6g (0.5mol) of (R)-N-benzyl-3-hydroxy piperidine and 50g (1.25mol) of sodium hydroxide into a 1000ml three-mouth flask; raising the temperature to 190°C for reaction for 15h; after HPLC monitoring and basic racemization, cooling the reaction liquid to about 50°C; adding water by drops and dissolving the product through methyl tertiary butyl ether (MTBE) feeding; cooling the reaction liquid to ambient temperature; separating the liquid and extracting the aqueous phase for one time with MTBE supplement; combing the organic phase and washing it with a small amount of water; obtaining 96g of racemized N-benzyl-3-hydroxy piperidine with a yield of 100% after concentration. (HPLC: R/S=51.9/48.1).

### Embodiment 2 Racemization of (R)- N-benzyl-3-hydroxy piperidine (II)

Adding 95.6g (0.5mol) of (R)-N-benzyl-3-hydroxy piperidine and 75g (1.27mol) of 90% potassium hydroxide into a 1000ml three-mouth flask; raising the temperature to 180°C for reaction for 15h; after HPLC monitoring and basic racemization, cooling the reaction liquid to about 50°C; adding water by drops and dissolving the product through MTBE feeding; cooling the reaction liquid to ambient temperature; separating the liquid and extracting the aqueous phase for one time with MTBE supplement; combing the organic phase and washing it with a small amount of water; obtaining 96g of racemized N-benzyl-3-hydroxy piperidine with a yield of 100% after concentration. (HPLC: R/S=50.8/49.2).

### Embodiment 3 Racemization of (R)- N-benzyl-3-hydroxy piperidine (III)

Adding 95.6g (0.5mol) of (R)-N-benzyl-3-hydroxy piperidine and 84g (2 mol) of lithium hydroxide into a 1000ml three-mouth flask; raising the temperature to 200°C for reaction for 15h; after HPLC monitoring and basic racemization, cooling the reaction liquid to about 50°C; adding water by drops and dissolving the product through MTBE feeding; cooling the reaction liquid to ambient temperature; separating the liquid and extracting the aqueous phase for one time with MTBE supplement; combing the organic phase and washing it with a small amount of water; obtaining 96g of racemized N-benzyl-3-hydroxy piperidine with a yield of 100% after concentration. (HPLC: R/S=52.1/47.9).

### Embodiment 4 Racemization of (S)- N-benzyl-3-hydroxy piperidine

Adding 95.6g (0.5mol) of (S)-N-benzyl-3-hydroxy piperidine and 75g (1.27mol) of 90% potassium hydroxide into a 1000ml three-mouth flask; raising the temperature to 180°C for reaction for 15h; after HPLC monitoring and basic racemization, cooling the reaction liquid to about 50°C; adding water by drops and dissolving the product through MTBE feeding; cooling the reaction liquid to ambient temperature; separating the liquid and extracting the aqueous phase for one time with MTBE supplement; combing the organic phase and washing it with a small amount of water; obtaining 96g of racemized N-benzyl-3-hydroxy piperidine with a yield of 100% after concentration. (HPLC: S/R=50.6/49.4).

### Embodiment 5 Racemization of recycled N-benzyl-3-hydroxy piperidine

Adding 95.6g (0.5mol; R/S=10/90) of resolved and recycled R/S-N-benzyl-3-hydroxy piperidine and 75g (1.27mol) of 90% potassium hydroxide into a 1000ml three-mouth flask; raising the temperature to 180°C for reaction for 15h; after HPLC monitoring and basic racemization, cooling the reaction liquid to about 50°C; adding water by drops and dissolving the product through MTBE feeding; cooling the reaction liquid to ambient temperature; separating the liquid and extracting the aqueous phase for one time with MTBE supplement; combing the organic phase and washing it with a small amount of water; obtaining 96g of racemized N-benzyl-3-hydroxy piperidine with a yield of 100% after concentration. (HPLC: S/R=50.4/49.6).

### Embodiment 6

Adding 95.6g (0.5mol; R/S=30/70) of resolved and recycled R/S-N-benzyl-3-hydroxy piperidine and 75g (1.27mol) of 90% potassium hydroxide into a 1000ml stainless steel autoclave; raising the temperature to 300°C for reaction for 1h; after HPLC monitoring and basic racemization, cooling the reaction liquid to about 50°C; adding water by drops and dissolving the product through MTBE feeding; cooling the reaction liquid to ambient temperature; separating the liquid and extracting the aqueous phase for one time with MTBE supplement; combing the organic phase and washing it with a small amount of water; obtaining 96g of racemized N-benzyl-3-hydroxy piperidine with a yield of 100% after concentration. (HPLC: S/R=50.5/49.5).

### Embodiment 7

Adding 95.6g (0.5mol; R/S=30/70) of resolved and recycled R/S-N-benzyl-3-hydroxy piperidine and 75g (1.27mol) of 90% potassium hydroxide into a 1000ml three-mouth flask; raising the temperature to 120°C for reaction for 100h; after HPLC monitoring and basic racemization, cooling the reaction liquid to about 50°C; adding water by drops and dissolving the product through MTBE feeding; cooling the reaction liquid to ambient temperature; separating the liquid and extracting the aqueous phase for one time with MTBE supplement; combing the organic phase and washing it with a small amount of water; obtaining 96g of racemized N-benzyl-3-hydroxy piperidine with a yield of 100% after concentration. (HPLC: S/R=51.5/48.5).

The above displays and describes the basic principle, main features and advantages of the Invention. A person skilled in the art shall understand that the Invention is not limited to the above embodiments. The above embodiments and the description in the Specification only explain the principle of this invention. Under the premise of not departing from the scope of the Invention, any changes and improvements of the Invention still fall within the protection scope of the Invention. The protective scope of the Invention is defined by the attached Claims.

## Claims

1. A method for racemization recycling of chiral N-benzyl-3-hydroxy piperidine, wherein, it uses chiral 1-benzyl-3-hydroxy piperidine as the raw material to generate racemized products through racemization reaction under the action of strong alkali and high temperature.

2. The method according to Claim 1, wherein, the said chiral N-benzyl-3-hydroxy piperidine is (R)-N-benzyl-3-hydroxy piperidine, (S)-N-benzyl-3-hydroxy piperidine or a mixture of the two.

3. The method according to Claim 2, wherein, the ee value scope of (R)-N-benzyl-3-hydroxy piperidine and (S)- N-benzyl-3-hydroxy piperidine in the mixture is 0-100%.

4. The method according to Claim 1 or 2, wherein, the said strong alkali comprises sodium hydroxide, potassium hydroxide or lithium hydroxide.

5. The method according to Claim 1 or 2, wherein, the temperature for the said racemization reaction is 120-300°C.

6. The method according to Claim 5, wherein, the temperature for the said racemization reaction is 180-200°C.

7. The method according to Claim 1 or 2, wherein, the time for the said racemization reaction is 1-100 hours.

8. The method according to Claim 7, wherein, the time for the said racemization reaction is 15 hours.

9. The method according to Claim 1 or 2, wherein, the mole ratio between the said chiral N-benzyl-3-hydroxy piperidine and the strong alkali is 1:1- 1:5, preferably 1:2.5-1:4.

10. The method according to Claim 1 or 2, wherein, it also comprises post-processing processes of the racemization reaction as: cooling the reaction liquid to 50°C after racemization reaction; adding water by drops for dilution; dissolving the product through MTBE feeding; cooling the reaction liquid to ambient temperature; separating the liquid; extracting the aqueous phase for one time with MTBE supplement; combing the organic phase and washing it with a small amount of water; obtaining racemized N-benzyl-3-hydroxy piperidine after concentration.

## Patentansprüche

1. Verfahren zum Racemisierungs-Recycling von chiralem N-Benzyl-3-Hydroxypiperidin, wobei chirales 1-Benzyl-3-Hydroxypiperidin als Ausgangsmaterial verwendet wird, um racemisierte Produkte durch Racemisierungsreaktion unter Einwirkung von starkem Alkali und hoher Temperatur zu erzeugen.

2. Verfahren nach Anspruch 1, wobei das chirale N-Benzyl-3-Hydroxypiperidin (R)-N-Benzyl-3-Hydroxypiperidin, (S)-N-Benzyl-3-Hydroxypiperidin oder eine Mischung der beiden ist.

3. Verfahren nach Anspruch 2, wobei der ee-Wertebereich von (R)-N-Benzyl-3-Hydroxypiperidin und (S)-N-Benzyl-3-Hydroxypiperidin in der Mischung 0-100% beträgt.

4. Verfahren nach Anspruch 1 oder 2, wobei das starke Alkali Natriumhydroxid, Kaliumhydroxid oder Lithiumhydroxid umfasst.

5. Verfahren nach Anspruch 1 oder 2, wobei die Temperatur für die Racemisierungsreaktion 120 bis 300°C beträgt.

6. Verfahren nach Anspruch 5, wobei die Temperatur für die Racemisierungsreaktion 180 bis 200°C beträgt.

7. Verfahren nach Anspruch 1 oder 2, wobei die Zeit für die Racemisierungsreaktion 1-100 Stunden beträgt.

8. Verfahren nach Anspruch 7, wobei die Zeit für die Racemisierungsreaktion 15 Stunden beträgt.

9. Verfahren nach Anspruch 1 oder 2, wobei das Molverhältnis zwischen dem chiralen N-Benzyl-3-Hydroxypiperidin und dem starken Alkali 1:1 bis 1:5, vorzugsweise 1:2,5 bis 1:4 beträgt.

10. Verfahren nach Anspruch 1 oder 2, wobei es auch Nachbearbeitungsprozesse der Racemisierungsreaktion umfasst: Abkühlen der Reaktionsflüssigkeit auf 50°C nach der Racemisierungsreaktion; Zugabe von Wasser zur Verdünnung; Auflösen des Produkts durch MTBE-Zuführung; Abkühlen der Reaktionsflüssigkeit auf Umgebungstemperatur; Trennen der Flüssigkeit; einmaliges Extrahieren der wässrigen Phase mit MTBE-Supplement; Kämmen der organischen Phase und Waschen mit einer kleinen Menge Wasser; Erhalten von racemisiertem N-Benzyl-3-Hydroxypiperidin nach dem Einengen.

## Revendications

1. Procédé destiné à la racémisation du recyclage de N-benzyl-3-hydroxy pipéridine chirale, dans lequel, il utilise 1-benzyl-3-hydroxy pipéridine chirale comme matières premières pour produire des produits racémisés par l'intermédiaire de la réaction de racémisation sous l'action d'un fort alcali et de la haute température.

2. Procédé selon la revendication 1, dans lequel, ladite N-benzyl-3-hydroxy pipéridine chirale est (R)-N-benzyl-3-hydroxy pipéridine, (S)-N-benzyl-3-hydroxy pipéridine ou un mélange des deux.

3. Procédé selon la revendication 2, dans lequel, l'étendue de la valeur ee de (R)-N-benzyl-3-hydroxy pipéridine et de (S)-N-benzyl-3-hydroxy pipéridine dans le mélange est de 0 à 100 %.

4. Procédé selon la revendication 1 ou 2, dans lequel, ledit fort alcali comprend l'hydroxyde de sodium, l'hydroxyde de potassium ou l'hydroxyde de lithium.

5. Procédé selon la revendication 1 ou 2, dans lequel la température destinée à la réaction de racémisation est de 120 à 300 °C.

6. Procédé selon la revendication 5, dans lequel la température destinée à la réaction de racémisation est de 180 à 200 °C.

7. Procédé selon la revendication 1 ou 2, dans lequel la durée destinée à la réaction de racémisation est 1 à 100 heures.

8. Procédé selon la revendication 7, dans lequel la durée destinée à la réaction de racémisation est 15 heures.

9. Procédé selon la revendication 1 ou 2, dans lequel, le rapport molaire entre ledit N-benzyl-3-hydroxy pipéridine chirale et le fort alcali est de 1:1 à 1:5, de préférence 1:2,5 à 1:4.

10. Procédé selon la revendication 1 ou 2, dans lequel, il comprend également les processus de post-traitement de la réaction de racémisation comme : le refroidissement du liquide de réaction à 50 °C après la réaction de racémisation ; l'ajout d'eau par gouttes pour la dilution ; la dissolution du produit par l'intermédiaire de l'alimentation de MTBE ; le refroidissement du liquide de réaction à température ambiante ; la séparation du liquide ; l'extraction de la phase aqueuse pour une fois avec le supplément de MTBE ; la combinaison de la phase organique et son lavage avec une petite quantité d'eau ; l'obtention de N-benzyl-3-hydroxy pipéridine racémisée après la concentration.
